# EUROPÄISCHE PATENTSCHRIFT

(11) **EP 3 628 279 B1**
(45) Veröffentlichungstag und Bekanntmachung des Hinweises auf die Patenterteilung: **06.11.2024**
(21) Anmeldenummer: 18196567.4
(22) Anmeldetag: 25.09.2018
(51) Int. Cl.: A61F 7/00, B05B 1/00, A61M 11/00, A61M 19/00

(54) **VORRICHTUNG ZUR ÖRTLICHEN BETÄUBUNG**
DEVICE FOR LOCAL NUMBING
DISPOSITIF D'ANESTHÉSIE LOCALE

(43) Veröffentlichungstag der Anmeldung: 01.04.2020
(73) Patentinhaber: Rotstein, Raphael, 80797 München (DE)
(72) Erfinder: Rotstein, Raphael, 80797 München (DE); Sturmes, Joachim, 85304 Ilmmünster (DE); Wolf, Alexa, 81925 München (DE)
(74) Vertreter: Farago-Schauer, Peter Andreas

(56) Entgegenhaltungen:
- CN-Y- 201 230 995
- GB-A- 2 545 884
- US-B1- 6 226 996

## Beschreibung

Die vorliegende Erfindung betrifft eine Vorrichtung zur örtlichen Betäubung eines Körperteils, bei der man die betreffenden Patienten durch Kälte, welche die Nervenzellfortsätze und damit die Nervenfunktion für eine gewisse Zeit beeinträchtigt, schmerzunempfindlich macht. Dabei wird erfindungsgemäß durch eine Austrittsöffnung der Vorrichtung ein Kältemittel, vorzugsweise medizinische, flüssige Kohlensäure, auf eine zu behandelnde Stelle eines Körperteils geleitet.

### Stand der Technik

Grundsätzlich basiert die Erfindung auf der Kryotherapie, wobei der Ausdruck "Kryotherapie" die Behandlung von verschiedenen Leiden durch oberflächliche Einwirkung großer Kälte bezeichnet. Die Kryotherapie führt bekanntermaßen zu verschiedenen physiologischen Wirkungen, z.B. der Heraufsetzung der Schmerzschwelle, einer Verminderung der Spastik, einer Entspannung von Muskelfasern oder auch einer blutstillenden Wirkung.

Im Stand der Technik wurde früher für die Kryotherapie insbesondere verflüssigter Stickstoff benutzt, der allerdings aufgrund der speziellen Bedingungen, unter denen er gelagert werden muss, schwere unhandliche Vorrichtungen benötigt. Aus den Vorrichtungen aus dem Stand der Technik mit verflüssigtem Stickstoff verdampfte der verflüssigte Stickstoff ständig, was ungewünschte Verluste verursachte. Außerdem ist flüssiger Stickstoff nur mit hohem Aufwand zu transportieren und großflächig bereitzustellen, was seinem Einsatz entsprechend entgegensteht.

Es ist diesbezüglich beispielsweise aus der EP-0 633 008 B1 bekannt, statt Stickstoff verflüssigtes Kohlendioxyd zu verwenden, das aus einer Druckflasche entnommen wird und über eine druckfeste Leitung zu einer pistolenartigen Vorrichtung geführt wird, in der es entspannt und dann auf eine zu behandelnde Stelle ausgestoßen wird. Dabei kann das ausgestoßene Gas Temperaturen von bis zu -75 °C erreichen, wodurch die hiermit behandelte Haut innerhalb weniger Sekunden von ihrer Normaltemperatur (ca. 32 °C) heruntergekühlt werden kann auf Temperaturen von 0 bis 5 °C.

Es hat sich jedoch dabei herausgestellt, dass der entsprechende Thermoschock z. B. zu einer neuroreflektorischen Ausdehnung der Gefäße im betroffenen Bereich führt, wodurch Entzündungsmediatoren schneller abtransportiert werden.

Zusätzlich beschreibt GB2545884A eine Zyklon-Düse zur Verabreichung verschiedener Substanzen, beispielsweise Sonnencreme, Body-Lotion, dermatologische Cremen, Wasser oder Kühlmittel.

### Zusammenfassung der Erfindung

Aufgabe der vorliegenden Erfindung ist es nun, eine Vorrichtung der beschriebenen Art dahingehend zu verbessern, dass eine zuverlässige und günstige Kühlung an einer zu behandelnden Stelle eines Körperteils erzielt wird.

Eine weitere Aufgabe der vorliegenden Erfindung ist es, eine Vorrichtung zu schaffen, bei der es nicht zu einer Kälteverbrennung der Dermis an einer zu behandelnden Stelle eines Körperteils kommen kann.

Die obigen Aufgaben und weitere der nachstehenden Beschreibung zu entnehmende Aufgaben werden durch eine Vorrichtung gemäß Hauptanspruch 1 gelöst.

Weitere vorteilhafte Ausgestaltungen und Ausführungsformen der vorliegenden Erfindung sind in den Unteransprüchen angegeben.

Bevorzugte Ausführungsbeispiele sind in der nachfolgenden Zeichnung und in einer detaillierten Beschreibung dargestellt, sie sollen aber die vorliegende Erfindung nicht ausschließlich darauf begrenzen.

Dabei zeigt:

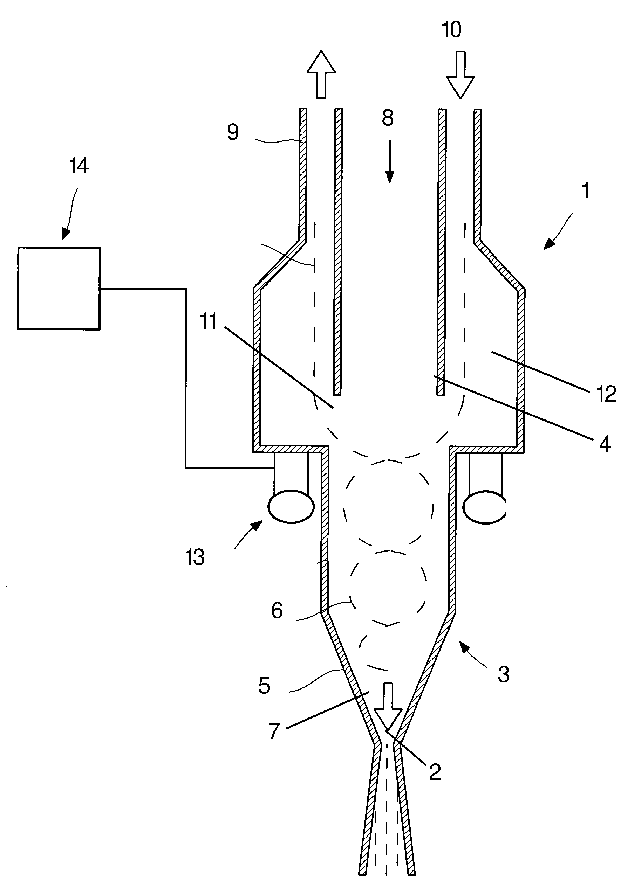

Fig. 1 eine schematische Ansicht einer Ausbildungsform einer Vorrichtung zur örtlichen Betäubung einer zu behandelnden Stelle eines Körperteils durch Kältemittel, insbesondere Gas. Detaillierte Beschreibung von Ausführungsbeispielen

Unter Bezugnahme auf die Fig. 1 wird eine Vorrichtung 1 zur örtlichen Betäubung mit einer Austrittsöffnung 2 gezeigt, durch die Kältemittel, insbesondere Gas, auf eine zu behandelnde Stelle geleitet wird, wobei die Vorrichtung 1 eine mit der Austrittsöffnung 2 verbundene Zyklon-Düse 3 aufweist, aus der das Kältemittel auf eine (nicht gezeigte) zu behandelnde Stelle eines (ebenfalls nicht gezeigten) Körperteils ausströmt. Das Kältemittel kann medizinische, flüssige Kohlensäure sein. Die Zyklon-Düse 3 hat den Vorteil, dass durch das in Rotation versetzte Gas eine wesentlich schnellere Abkühlung des Gewebes erreicht wird, was zu einer deutlich kürzeren Behandlungszeit führt.

Die Austrittsöffnung 2 ist vorzugsweise als Austrittsdüse mit einem stromabwärts trichterförmig aufgeweiteten Ende ausgebildet. Die Trichterform der Austrittsöffnung 2 ermöglicht es, den Kältemittelstrahl an der zu behandelnden Stelle mehr oder weniger zu fokussieren.

Die Zyklon-Düse 3 weist ein stromaufwärts gelegenes zylindrisches Segment 4 und ein daran anschließendes stromabwärts gelegenes kegelstumpfförmiges Segment 5 auf.

Darüber hinaus weist die Vorrichtung 1 Mittel 8 zur tangentialen Einführung des Kältemittels in das Innere des zylindrischen Segments 4 auf, wobei durch den tangentialen Eintritt in das Innere des zylindrischen Segments 4 das Kältemittel auf eine Kreisbahn 6 gezwungen wird und somit in einem abwärts gerichteten Wirbel nach unten strömt. Die Mittel 8 zur tangentialen Einführung des Kältemittels in das Innere des zylindrischen Segments 4 umfassen vorzugsweise eine Gasdruckflasche, was die Mobilität der Vorrichtung gestattet. Die stromabwärts vom Segment 4 gelegene Verjüngung des kegelstumpfförmigen Segments 5 bewirkt eine Volumen-Verdrängung nach innen und ein Aufstauen im am weitesten stromabwärts gelegenen Bereich 7 des kegelstumpfförmigen Segments 5.

Die Zyklon-Düse 3 weist weiterhin eine äußere Ummantelung 9 auf, die sich um das zylindrische Segment 4 erstreckt. Die

Vorrichtung umfasst Mittel 10 zur Einführung von Druckluft in den Raum zwischen der äußeren Ummantelung 9 und dem zylindrischen Segment 4, wobei das zylindrische Segment 4 mit mindestens einer Öffnung (11) versehen ist, um die Vermischung der Druckluft und des Kältemittels zu gestatten. Die Mittel 10 zur Einführung von Druckluft in den Raum zwischen der äußeren Ummantelung 9 und dem zylindrischen Segment 4 umfassen beispielsweise eine Druckluftflasche, was die Mobilität der Vorrichtung gestattet.

Die äußere Ummantelung 9 hat eine geringere axiale Erstreckung als das zylindrische Segment 4, und vorzugsweise weist die äußere Ummantelung 9 in ihrem stromabwärts gelegenen Bereich 12 eine Erweiterung auf, um eine Expansionskammer für die Druckluft zu bilden.

Die Vorrichtung 1 weist weiterhin einen berührungslosen Temperatursensor 13 auf, wobei der berührungslose Temperatursensor 13 angeordnet ist, um die Temperatur an der zu behandelnden Stelle zu erfassen.

Weiterhin weist die Vorrichtung 1 eine Steuerung 14 auf, die operativ mit dem berührungslosen Temperatursensor 13 verbunden ist und ausgebildet ist, um die Vorrichtung 1 beim Unterschreiten einer Zieltemperatur an der zu behandelnden Stelle abzuschalten und/oder ein Warnsignal zu erzeugen, wobei vorzugsweise das Warnsignal ein optisches und/oder akustisches Signal ist. Dadurch ist erfindungsgemäß eine automatische und/oder eine manuelle Abschaltung der Vorrichtung zur Vermeidung der Beschädigung der Dermis an der zu behandelnden Körperstelle möglich.

Die Steuerung kann auch ausgebildet sein, um den Betrieb der Vorrichtung 1 zur Einhaltung der Zieltemperatur an der zu behandelnden Stelle automatisch zu regeln.

Darüber hinaus ist es denkbar, die Zeitdauer der Zieltemperatur an der zu behandelnden Körperstelle über die Steuerung 14 einzustellen bzw. zu begrenzen, um die Sicherheit der Vorrichtung zur Vermeidung von Kälteverbrennungen zu vermeiden. In diesem Zusammenhang ist es erfindungsgemäß denkbar, vorprogrammierte Zieltemperatur-Zeitdauer-Paare in der Steuerung 14 abzuspeichern und diese Paare benutzerabhängig abzurufen.

Der berührungslose Temperatursensor 13 kann vorzugsweise als Pyrometer oder als Infrarot-Temperatursensor ausgebildet sein.

Vorzugsweise ist die Steuerung 14 ausgebildet, um die Zieltemperatur frei einzustellen, wobei die Zieltemperatur vorzugsweise höher als 0°C ist.

Nach einem weiteren bevorzugten Aspekt weist die Vorrichtung 1 weiter einen Laser-Pointer 15 auf, der ausgebildet und an der Vorrichtung 1 positioniert ist, um die Ausrichtung des aus der Vorrichtung 1 austretenden Kältemittels mit der zu behandelnden Stelle zu gestatten. Somit wird dem Benutzer der Vorrichtung 1 ermöglicht, die Austrittsdüse zuverlässig und einfach auf die zu behandelnde Stelle zu richten. Für die zuverlässige und leichte Ausrichtung der Vorrichtung ist vorzugsweise zumindest die Austrittsöffnung 2 oder die Zyklon-Düse 3 als Hand-Vorrichtung ausgebildet.

Die Vorrichtung 1 kann weiterhin vorzugsweise einen um die Austrittsöffnung 2 angeordneten Schalldämpfer 16 umfassen.

Weitere mögliche Ausbildungsformen sind in den folgenden Ansprüchen beschrieben.

Insbesondere können auch die verschiedenen Merkmale der oben beschriebenen Ausfiihrungsformen miteinander kombiniert werden, soweit sie sich nicht technisch ausschließen.

Die in den Ansprüchen genannten Bezugszeichen dienen nur der besseren Verständlichkeit und beschränken die Ansprüche in keiner Weise auf die in den Figuren dargestellten Formen.

## Patentansprüche

1. Vorrichtung (1) zur örtlichen Betäubung mit einer Austrittsöffnung (2), durch die Kältemittel, insbesondere Gas, auf eine zu behandelnde Stelle geleitet wird,
wobei die Vorrichtung (1) eine mit der Austrittsöffnung (2) verbundene Zyklon-Düse (3) aufweist, aus der das Kältemittel auf eine zu behandelnde Stelle ausströmt, wobei die Zyklon-Düse (3) ein stromaufwärts gelegenes zylindrisches Segment (4) und ein daran anschließendes stromabwärts gelegenes kegelstumpfförmiges Segment (5) umfasst und dass die Vorrichtung (1) weiterhin Mittel (8) zur tangentialen Einführung des Kältemittels in das Innere des zylindrischen Segments (4) umfasst, wobei durch den tangentialen Eintritt in das Innere des zylindrischen Segments (4) das Kältemittel auf eine Kreisbahn (6) gezwungen wird und somit in einem abwärts gerichteten Wirbel nach unten strömt, und wobei die Verjüngung des kegelstumpfförmigen Segments (5) eine Volumen-Verdrängung nach innen und einen Aufstau im am weitesten stromabwärts gelegenen Bereich (7) des kegelstumpfförmigen Segments (5) bewirkt, **dadurch gekennzeichnet, dass**
die Zyklon-Düse (3) eine äußere Ummantelung (9) umfasst, die sich um das zylindrische Segment (4) erstreckt, und dass die Vorrichtung weiter Mittel (10) zur Einführung von Druckluft in den Raum zwischen der äußeren Ummantelung (9) und dem zylindrischen Segment (4) umfasst, wobei das zylindrische Segment (4) mit mindestens einer Öffnung (11) versehen ist, um die Vermischung der Druckluft und des Kältemittels zu gestatten.

2. Vorrichtung (1) gemäß Anspruch 1, **dadurch gekennzeichnet, dass** die Austrittsöffnung (2) als Austrittsdüse ausgebildet ist.

3. Vorrichtung (1) gemäß Anspruch 1 oder 2, **dadurch gekennzeichnet, dass** die äußere Ummantelung (9) eine geringere axiale Erstreckung als das zylindrische Segment (4) hat und/oder dass die äußere Ummantelung (9) in ihrem stromabwärts gelegenen Bereich (12) eine Erweiterung aufweist.

4. Vorrichtung (1) gemäß einem oder mehreren der vorangehenden Ansprüche 1 bis 3, **dadurch gekennzeichnet, dass** die Vorrichtung (1) einen berührungslosen Temperatursensor (13) umfasst, wobei der berührungslose Temperatursensor (13) angeordnet ist, um die Temperatur an der zu behandelnden Stelle zu erfassen, und dass die Vorrichtung (1) weiterhin eine Steuerung (14) umfasst, die operativ mit dem berührungslosen Temperatursensor (13) verbunden und ausgebildet ist, um die Vorrichtung (1) beim Unterschreiten einer Zieltemperatur an der zu behandelnden Stelle abzuschalten und/oder ein Warnsignal zu erzeugen, wobei vorzugsweise das Warnsignal ein optisches und/oder akustisches Signal ist.

5. Vorrichtung (1) gemäß einem oder mehreren der vorangehenden Ansprüche 1 bis 3, **dadurch gekennzeichnet, dass** die Vorrichtung (1) einen berührungslosen Temperatursensor (13) umfasst, wobei der berührungslose Temperatursensor (13) angeordnet ist, um die Temperatur an der zu behandelnden Stelle zu erfassen, und dass die Vorrichtung (1) weiterhin eine Steuerung (14) umfasst, die operativ mit dem berührungslosen Temperatursensor (13) verbunden und ausgebildet ist, um den Betrieb der Vorrichtung (1) zur Einhaltung einer Zieltemperatur an der zu behandelnden Stelle zu regeln.

6. Vorrichtung gemäß Anspruch 4 oder 5, **dadurch gekennzeichnet, dass** der berührungslose Temperatursensor (13) als Pyrometer oder als Infrarot-Temperatursensor ausgebildet ist.

7. Vorrichtung (1) gemäß einem oder mehreren der vorangehenden Ansprüche 4 bis 6, **dadurch gekennzeichnet, dass** die Steuerung (14) ausgebildet ist, um die Zieltemperatur frei einzustellen, wobei die Zieltemperatur vorzugsweise höher als 0°C ist.

8. Vorrichtung (1) gemäß einem oder mehreren der vorangehenden Ansprüche 1 bis 7, **dadurch gekennzeichnet, dass** die Vorrichtung (1) weiter einen Laser-Pointer (15) umfasst, der ausgebildet und an der Vorrichtung (1) positioniert ist, um die Ausrichtung des aus der Vorrichtung (1) austretenden Kältemittels mit der zu behandelnden Stelle zu gestatten.

9. Vorrichtung (1) gemäß einem oder mehreren der vorangehenden Ansprüche 1 bis 8, **dadurch gekennzeichnet, dass** die Vorrichtung (1) weiter einen um die Austrittsöffnung (2) angeordneten Schalldämpfer (16) umfasst.

10. Vorrichtung (1) gemäß einem oder mehreren der vorangehenden Ansprüche 1 bis 9, **dadurch gekennzeichnet, dass** das Kältemittel medizinische, flüssige Kohlensäure ist.

11. Vorrichtung (1) gemäß einem oder mehreren der vorangehenden Ansprüche 1 bis 9, **dadurch gekennzeichnet, dass** die Vorrichtung (1) mindestens teilweise als Handgerät ausgebildet ist.

## Claims

1. Device (1) for local anesthesia having an outlet opening (2) through which coolant, in particular gas, is directed at a site to be treated,
the device (1) having a cyclone nozzle (3) connected with the outlet opening (2) from which the coolant flows out towards a site to be treated, the cyclone nozzle (3) having an upstream cylindrical segment (4) and an adjoining downstream segment (5) in the form of a truncated cone, the device (1) further comprising means (8) for the tangential introduction of the coolant into the interior of the cylindrical segment (4); the coolant being forced into a circular path (6) by the tangential introduction into the interior of the cylindrical segment (4) and consequently flowing downwards in an eddy directed downward; and the tapering of the segment (5) having the form of a truncated cone effecting volume displacement towards the inside and banking in the region (7) of the segment (5) having the form a truncated cone which is furthest downstream; **characterized in that**
the cyclone nozzle (3) comprises an outer sheath (9) extending around the cylindrical segment (4), and **in that** the device further comprises means (10) for the introduction of compressed air into the space between the outer sheath (9) and the cylindrical segment (4); the cylindrical segment (4) being provided with at least one opening (11) so as to allow mixing of the compressed air and the coolant.

2. Device (1) according to Claim 1, **characterized in that** the outlet opening (2) is embodied as an outlet nozzle.

3. Device (1) according to Claim 1 or 2, **characterized in that** the outer sheath (9) has a lesser axial extension than the cylindrical segment (4), and/or **in that** the outer sheath (9) has an expansion in its downstream area (12).

4. Device (1) according to one or more of the above Claims 1 through 3, **characterized in that** the device (1) comprises a non-contact temperature sensor (13) which is arranged so as to detect the temperature at the site to be treated, and **in that** the device (1) further comprises a controller (14) which is operatively connected to the non-contact temperature sensor (13) and adapted to switch off the device (1) when falling below a target temperature at the site to be treated and/or to generate an alarm signal which is preferably an optical and/or an acoustic signal.

5. Device (1) according to one or more of the above Claims 1 through 3, **characterized in that** the device (1) comprises a non-contact temperature sensor (13), the non-contact temperature sensor (13) being arranged to detect the temperature at the site to be treated, and **in that** the device (1) further comprises a controller (14) which is operatively connected to the non-contact temperature sensor (13) and adapted to control operation of the device (1) to maintain a target temperature at the site to be treated.

6. Device according to Claim 4 or 5, **characterized in that** the non-contact temperature sensor (13) is embodied as a pyrometer or an infrared temperature sensor.

7. Device (1) according to one or more of the above Claims 4 through 6, **characterized in that** the controller (14) is adapted to freely set the target temperature, the target temperature being preferably higher than 0°C.

8. Device (1) according to one or more of the above Claims 1 through 7, **characterized in that** the device (1) further comprises a laser pointer (15) adapted and positioned at the device (1) so as to allow alignment of the coolant issuing from the device (1) with the site to be treated.

9. Device (1) according to one or more of the above Claims 1 through 8, **characterized in that** the device (1) further comprises a sound absorber (16) arranged around the outlet opening (2).

10. Device (1) according to one or more of the above Claims 1 through 9, **characterized in that** the coolant is medical liquid carbonic acid.

11. Device (1) according to one or more of the above Claims 1 through 9, **characterized in that** the device (1) is at least partly embodied as a hand-held device.

## Revendications

1. Dispositif (1) d'anesthésie locale comportant une ouverture de sortie (2) par laquelle un réfrigérant, en particulier un gaz, est dirigé vers un site à traiter, le dispositif (1) présentant une buse cyclone (3) reliée à l'ouverture de sortie (2) à partir de laquelle le réfrigérant s'écoule vers un site à traiter, la buse cyclone (3) comprenant un segment cylindrique amont (4) et un segment en forme de tronc conique aval (5) s'y raccordant, et le dispositif (1) présentant en outre des moyens (8) pour l'introduction tangentielle du réfrigérant à l'intérieur du segment cylindrique (4), le réfrigérant étant poussé sur une piste circulaire (6) par l'entrée tangentielle à l'intérieur du segment cylindrique (4) et s'écoulant ainsi vers le bas dans un tourbillon dirigé vers le bas, et le rétrécissement du segment cylindrique en forme de tronc conique (5) provoquant un refoulement de volume vers l'intérieur et une accumulation dans une autre zone située le plus loin en aval (7) du segment en forme de tronc conique (5), **caractérisé en ce que**
la buse cyclone (3) comprend une enveloppe extérieure (9) s'étendant autour du segment cylindrique (4), et que le dispositif comprend en outre des moyens (10) pour introduire de l'air comprimé dans l'espace entre l'enveloppe extérieure (9) et le segment cylindrique (4), le segment cylindrique (4) étant pourvu d'au moins une ouverture (11) permettant le mélange de l'air comprimé et du réfrigérant.

2. Dispositif (1) selon la revendication 1, **caractérisé en ce que** l'ouverture de sortie (2) est réalisée sous la forme d'une buse de sortie.

3. Dispositif (1) selon la revendication 1 ou 2, **caractérisé en ce que** l'enveloppe extérieure (9) présente une extension axiale inférieure à celle du segment cylindrique (4) et/ou **en ce que** l'enveloppe extérieure (9) présente une extension dans sa zone aval (12) .

4. Dispositif (1) selon une ou plusieurs des revendications précédentes 1 à 3, **caractérisé en ce que** le dispositif (1) comprend un capteur de température sans contact (13), le capteur de température sans contact (13) étant conçu pour détecter la température à l'endroit à traiter, et **en ce que** le dispositif (1) comprend en outre une commande (14) qui est connectée fonctionnellement au capteur de température sans contact (13) et est conçue pour arrêter le dispositif (1) en cas de chute en dessous d'une température cible à l'endroit à traiter et/ou pour générer un signal d'avertissement, le signal étant de préférence un signal optique et/ou acoustique.

5. Dispositif (1) selon une ou plusieurs des revendications précédentes 1 à 3, **caractérisé en ce que** le dispositif (1) comprend un capteur de température sans contact (13), le capteur de température sans contact (13) étant conçu pour détecter la température à l'endroit à traiter, et **en ce que** le dispositif (1) comprend en outre une commande (14) qui est reliée fonctionnellement au capteur de température sans contact (13) et est conçue pour commander le fonctionnement du dispositif (1) afin de maintenir une température cible à l'endroit à traiter.

6. Dispositif selon la revendication 4 ou 5, **caractérisé en ce que** le capteur de température sans contact (13) est réalisé sous la forme d'un pyromètre ou d'un capteur de température infrarouge.

7. Dispositif (1) selon une ou plusieurs des revendications précédentes 4 à 6, **caractérisé en ce que** la commande (14) est conçue pour régler librement la température cible, la température cible étant de préférence supérieure à 0°C.

8. Dispositif (1) selon une ou plusieurs des revendications précédentes 1 à 7, **caractérisé en ce que** le dispositif (1) comprend en outre un pointeur laser (15) qui est formé et positionné sur le dispositif (1) pour permettre l'alignement du réfrigérant s'échappant du dispositif (1) avec l'endroit à traiter.

9. Dispositif (1) selon une ou plusieurs des revendications précédentes 1 à 8, **caractérisé en ce que** le dispositif (1) comprend en outre un silencieux (16) disposé autour de l'ouverture de sortie (2).

10. Dispositif (1) selon une ou plusieurs des revendications précédentes 1 à 9, **caractérisé en ce que** le réfrigérant est du dioxyde de carbone liquide médical.

11. Dispositif (1) selon une ou plusieurs des revendications précédentes 1 à 9, **caractérisé en ce que** le dispositif (1) est réalisé au moins partiellement sous la forme d'un appareil début à main.
